# EUROPEAN PATENT SPECIFICATION

(11) **EP 4 380 658 B1**
(45) Date of publication and mention of the grant of the patent: **29.10.2025**
(21) Application number: 21762256.2
(22) Date of filing: 05.08.2021
(51) Int. Cl.: A61M 16/20, A61M 16/08

(54) **GAS VALVE FOR A VENTILATION APPARATUS**
GASVENTIL FÜR EIN VENTILATIONSGERÄT
SOUPAPE A GAZ POUR UN APPAREIL DE VENTILATION

(43) Date of publication of application: 12.06.2024
(73) Proprietor: ZOLL Medical Corporation, Chelmsford, MA 01824-4105 (US)
(72) Inventor: VARGA, Christopher M., Laguna Hills, California 92653 (US); HESS, Martin Björn, 7208 Malans (CH); VAN DER STAAY, Matthias, 8758 Obstalden (CH)
(74) Representative: Potter Clarkson
(86) International application number: PCT/US2021/044613
(87) International publication number: WO 2023/014356

(56) References cited:
- EP-A1- 0 552 916
- EP-A1- 3 243 046
- EP-B1- 3 243 046
- WO-A1-2013/164733
- WO-A1-2020/214490
- GB-A- 2 144 334

## Description

The present invention relates to a leakage gas valve for a ventilation apparatus.

During artificial respiration of a patient, it is desirable to prevent the patient from completely exhaling, and thereby prevent the patient's lungs from fully deflating during artificial respiration. This is because complete deflation, and subsequent reflation of the patient's lungs requires a significant amount of the patient's energy - in particular more than a partial deflation. Prevention of total exhalation is generally achieved by including a mechanism in the respiratory circuit which allows exhaled breath to escape the respiratory circuit through a gas valve.

Leakage gas valves, like exhalation valves, known in the state of the art can be used for breathing connections with two-way breathing to control the breathing gas. The exhalation air is directed to the ambient environment through the exhalation valve. When exhaling, the common exhalation valve only opens when the pressure of the exhaled gas (e.g. air) is greater than a preloaded pressure at the exhalation valve (e.g., > 4.2 mbar).

There are presently various forms of exhalation valves for respiratory breathing circuits.

EP 3 243 046 A1 discloses a bi-directional flow meter comprising a sampling tube through which fluid may flow and a sensor arrangement. The sampling tube comprises a first hollow section having a first internal cross-sectional area and a second hollow section having a second internal cross-sectional area being less than the first internal cross-sectional area. The sensor arrangement is for measuring the pressures within the constriction of the second hollow section. EP 0 552 916 A1 discloses another flow meter with a venturi nozzle.

The drawback of these solutions is, that those flow meters are not usable as a leakage gas valve.

WO 2013/164733 A1 discloses an exhalation valve system configured to control gas flow during exhalation of a subject. The valve is covered in a chamber and a housing cab and comprises a variable leak adjustment disk configured to control the gas leakage from subject interface. The variable leak adjustment disk is configured to engage a housing cap on the side of the housing cap that is opposite the chamber. The variable leak adjustment disk is configured such that a user may manually rotate the variable leak adjustment disk relative to the housing cap to align and/or partially align one or more variable leak orifices with one or more housing cap orifices to manually control the amount of gas leaked from valve.

The drawback of these solutions is, that the exhalation valve comprises a complicated structure with many rotatable elements, which must be controlled by a user.

Finally, WO2020/214490A1 discloses a gas valve with a release gas flow measurement arrangement.

An aim of the present invention is to avoid at least some of the drawbacks of the prior art, in particular, to provide an improved leakage gas valve to increase the leakage of a gas valve during exhalation, especially to support the exhalation effort of a patient. Furthermore, another aim of this invention is to provide a circuit comprising at least said gas valve for reducing the work of breathing of a patient during exhalation.

At least some of said aims are accomplished by a gas valve and a circuit for a ventilation system according to the independent claims.

The invention is defined by the appended claims. Subject matter referred as embodiments and /or disclosures which does not fall under the scope of the claims is provided for context and understanding.

A leakage gas valve for a ventilation apparatus according to a first embodiment of the invention comprises a first valve body having a main gas path chamber for guiding an inhalation gas to a patient, comprising at least three chamber sections (first, second and third chamber section), an inlet duct for supplying the ventilation gas to the first chamber section and an outlet duct for releasing the ventilation gas from the third chamber section. Said first chamber section and said third chamber section comprise a first cross-section area and said second chamber section comprises a second cross-section area, wherein said second cross-section area is smaller than said first cross-section area. Said second chamber section comprises a leakage channel for allowing a leakage gas flow out of the gas valve during exhalation.

Such a gas valve can be used as a leakage gas valve for an artificial ventilation or respiration system, in particular configured to ventilate a patient. Said second cross-section area is smaller than said first cross-section area, thus a negative gas pressure is created in the second cross-section area by the venturi effect, which prevents gas from leaking out of the leakage channel during inhalation or may even draw a small amount of ambient gas into the gas valve through the leakage channel during inhalation. Said first, second and third cross-section areas are cross-sections in the main gas path passage. Said cross section areas are flow cross-sectional areas for the ventilation gas passage through the, preferably tubular, leakage gas valve.

During inhalation the ventilation gas is directed from the artificial ventilation apparatus through the three cross-section areas to the patient and a dynamic gas pressure is generated in the three chamber sections due to the different cross sections - the venturi principle occurs within the main ventilation path of the gas valve. Hence the differential pressure between the main ventilation path in the gas valve and the ambient is reduced, which leads to a minimized leakage flow to the ambient through said leakage channel during inhalation. During exhalation, the gas flow from the ventilation apparatus to the patient is zero or reduced to a minimum. Hence no venturi effect occurs in said three chamber sections of said gas valve and the leakage gas flow out of the leakage channel of the gas valve is higher. Thus, the work of breathing during exhalation is reduced and therefore does not cause a resistance to the patient exhalation effort. In other words, a gas valve with a leakage channel in the main ventilation path, as described above, deactivates or weakens the leakage gas flow during inhalation by a gas pressure reduction in the main ventilation path and activates or increases the leakage gas flow during exhalation. Thus, the leakage only occurs during exhalation, which conserves ventilation gas (e.g. oxygen) and reduces the needed ventilation power of the ventilator apparatus. Furthermore, a simplified gas valve is provided, where the leakage is purely controlled by the ventilation gas flow and the properties/geometry of the three chamber sections.

Preferably said leakage channel comprises a controllable valve for controlling said leakage gas flow. Said controllable valve is placed in the second chamber section and comprises a very low opening pressure valve. The action of the negative pressure created during inhalation may keep or may pull the controllable valve mechanically closed. During exhalation it may open very easily for low work of breathing or low exhalation effort of the patient. Said controllable valve is preferably a gate-type valve, a flapper valve, an umbrella valve or a mushroom valve etc. Such controllable valves do not allow gas flow to come into the gas valve from ambient during inhalation.

Preferably said leakage channel is a tubular channel, thus the leakage gas flow during exhalation can be easily controlled and can be measured reproducibly.

Preferably said leakage channel contains at least a venturi valve, comprising a venturi nozzle, which is used to measure the leakage gas flow during exhalation according to the principles of a venturi flowmeter concept, wherein pressure can be measured at two locations within the venturi nozzle and can be used to calculate the leakage gas flow rate during exhalation based of the geometric structure of the venturi nozzle in the leakage channel.

Preferably said leakage channel is arranged at a minimum cross section area at said second chamber section. During inhalation the venturi effect is not disturbed and during exhalation the leakage gas flow is optimized.

Preferably a measuring device for measuring said leakage gas flow of said leakage channel during exhalation is provided. Said measuring device may use a pressure differential measurement sensor, especially a pressure differential measurement sensor of said ventilator apparatus. Said measuring device detects the exhalation behaviour of the patient during exhalation. The measured data may be used to accurately control the ventilation apparatus during ventilation, when said measuring device is connected to a controller of said ventilator apparatus.

Preferably said leakage channel comprises at least one opening for connecting said measuring device for measuring said leakage gas flow. Said at least one opening may be connected with connections for measuring the pressure or the differential pressure in the leakage channel to determine the gas flow through the leakage channel during inhalation and/or exhalation.

Preferably said leakage channel comprises at least a filter medium for filtering said leakage gas flow to filter inhaled and/or exhaled ventilation gas. Filtering the inhaled ventilation gas may reduce or prevent patient exposure to e.g. bacteria and filtering the exhaled ventilation gas may reduce or prevent exposure of the ambient environment to e.g. bacteria of the exhalation gas from the patient.

Preferably the second cross-section area of the second chamber section is adjustable. As described above a venturi principle occurs within the main ventilation path of the gas valve. Adjusting the second cross-section area of the second chamber section is useful to match the gas flow rate in the main gas path by adjusting the venturi nozzle geometry in the main gas path. Furthermore, by adjusting the second cross-section area an on-state and off-state of the venturi effect is provided to either produce a gas pressure reduction in the main gas path or not.

Preferably said second chamber section comprises an adjustable chamber wall. The thickness of said chamber wall may be increased or decreased or the location of said chamber wall may be changed to adjust the second cross-section area in the second chamber section to produce a desired venturi geometry in the second chamber section.

Preferably the second cross-section area of the second chamber section is adjustable and said second chamber section comprises at least a first cage structure with at least one adjustable balloon. The first cage structure forms the geometric structure of at least one side of the venturi geometry. Said at least adjustable balloon may be inflated to fill out the cage structure. In other words, said cage structure is a three-dimensional grid structure, forming a contained volume or shape (e.g. venturi nozzle) which can be filled by an expandable member such as a balloon. In addition, a second cage structure with at least another adjustable balloon may be arranged in the main gas path to form the geometric structure of the other side of the venturi geometry in the second gas chamber. A three-dimensional view of the gas valve provides that, the geometric structure formed by the cage structure or three-dimensional grid structure can be partitioned into one or more sections which can be filled by one or more balloon or inflatable members.

Preferably said adjustable balloon chamber comprising a controllable opening for connecting a fluid pump. Said adjustable balloons are connected to the said fluid pump, which inflate the balloons with a gas or a fluid. Furthermore, said balloons comprise a valve for deflating said balloons.

In a preferred embodiment, a second valve body is provided, comprising a bypass gas channel for receiving an inhalation gas, wherein said second valve body is connected to said first valve body by said individual leakage channel of the main gas path. Said second valve body may be connected to an individual ventilation gas supply and said ventilation gas may pass the leakage channel during inhalation and advantageously will support the patient's inhalation effort. Said second valve body may be branch-connected to a ventilator or actuator of said ventilation apparatus.

Preferably said second valve body comprises a venturi nozzle. Said venturi nozzle is adjacent to the main gas path and may control the leakage gas flow by activating or deactivating the controllable valve at a leakage channel opening that communicates between the main gas path and the bypass gas channel.

Furthermore, the venturi nozzle in the bypass gas channel may control leakage gas in the leakage channel by augmenting or increasing the pressure differential across an opening (e.g. orifice) that communicates between the main gas path and the bypass gas channel.

In particular said venturi nozzle may comprise a perpendicular or off-angle control flow path that can be turned on or off to switch/divert the bypass gas channel between at least two paths. A first path, which augments or enables leakage, and a second path, which reduces or prevents leakage gas flow.

Preferably the second chamber section in the second valve body comprises at least a second controllable leakage channel. Hence the at least second controllable leakage channel deactivates or weakens the leakage gas flow during inhalation by a gas pressure reduction in the bypass channel and activates the leakage gas flow during exhalation.

Furthermore, a circuit for a ventilation system comprising a ventilation limb and at least a gas valve disclosed herein and the gas valve is preferably assembled at the distal end of the ventilation limb. Said distal end of said ventilation limb, generally, is connected directly to a ventilator apparatus. Said circuit comprises said gas valve, whereby said circuit is connected via said gas valve directly to said ventilator apparatus. Therefore, said leakage channel can be easily connected to the ventilator apparatus via pressure pipes/tubes. Thus, short pressure pipes/tubes can be used during operation.

Furthermore, a circuit for a ventilation system is provided, comprising a ventilation limb and at least a gas valve as disclosed herein. Preferably the gas valve is assembled at the proximal end of the ventilation limb. Said proximal end of the ventilation limb is connected to a respiration mask or a respiration cannula for a patient for artificial respiration. Therefore, a standardized carbon dioxide concentration or carbon dioxide clearance can be enabled.

Preferably a carbon dioxide measurement is connected to the circuit, while the carbon dioxide measurement is advantageously assembled in the gas valve. Therefore, a carbon dioxide concentration or carbon dioxide clearance can be determined at said circuit or in said gas valve.

Advantageously said carbon dioxide measurement is a colorimetric carbon dioxide indicator. Therefore, said carbon dioxide concentration or carbon dioxide clearance performance can be visualized. Said colorimetric carbon dioxide indicator can be a strip, a grid and/or a graduated scale for easy visualization of said carbon dioxide concentration or carbon dioxide clearance performance with said gas valve.

A further gas valve for a ventilation apparatus according to another embodiment of the invention, comprises a first valve body having a main gas path chamber for guiding an inhalation gas to a patient, comprising at least three chamber sections (first, second and third chamber section), an inlet duct for supplying the ventilation gas to the first chamber section and an outlet duct for releasing the ventilation gas from the third chamber section. The second cross-section area of the second chamber section is adjustable. Hence, when it is desirable to have no leakage gas flow or to reduce leakage gas flow, the second cross-section area of the second chamber section is adjustable to form a venturi shape, which creates a negative pressure at the leakage channel. Said second cross-section area may be adjusted to form a venturi nozzle. Said adjustable venturi nozzle is arranged within the main gas path with the leakage channel located at the venturi minimum cross-sectional area.

Preferably said second chamber section comprises at least a first cage structure with at least a first adjustable balloon and preferably with a second adjustable balloon. The venturi geometry/shape is created/controlled either by the shape of the cage structure and/or by a perforated/open cage wall structure comprising holes or openings for pass through of said gas flow. Therefore, when the balloons are inflated, they will fill the cage structure to form the venturi shape in the main gas path and when they are deflated the gas can flow freely through the perforations or openings or holes in the venturi shaped wall in the main gas path. In other words, said cage structure is a three-dimensional grid structure, forming a contained volume or shape (e.g. venturi nozzle) which can be filled by an expandable member such, as a balloon.

Further preferably said first and second inflated balloons form a venturi nozzle in said second chamber section. In this embodiment, the venturi nozzle is created or controlled by inflation/deflation of said balloons or other compliant structures, like an adjustable wall, while no cage structure is needed. When it is desirable to have no leakage gas flow or to reduce leakage gas flow, the balloons are inflated to form the venturi shape which creates the negative gas pressure at the leakage channel. A three-dimensional view of the gas valve provides that, the geometric structure formed by the cage structure or three-dimensional grid structure can be partitioned into one or more sections which can be filled by one or more balloon or inflatable members.

Preferably said balloon is connected to a fluid pump. Said adjustable balloons are connected to the said fluid pump, which inflate the balloons with a gas or a fluid. Furthermore, said balloons comprise a valve for deflating said balloons.

Preferably said second chamber section comprises a leakage channel for allowing a leakage gas flow out of the gas valve during exhalation, as described in the gas valve embodiments above. During inhalation the ventilation gas is directed from the ventilation apparatus to the patient and a dynamic gas pressure is generated in the three chamber sections due to the different cross sections - venturi principle occurs within the main ventilation path of the gas valve. Hence the differential pressure between the main ventilation path in the gas valve and the ambient is reduced, which leads to a minimized leakage flow through said leakage channel during inhalation. During exhalation, the gas flow from the ventilation apparatus to the patient is zero or reduced to a minimum.

In another preferred embodiment another circuit for a ventilation system comprising a ventilation limb with a second valve body is provided. Said limb comprises a leakage opening, e.g. a hole and said second valve body comprising at least three chamber sections (first, second and third chamber section), an inlet duct for supplying the ventilation gas to the first chamber section and an outlet duct for releasing the ventilation gas from the third chamber section. Said first chamber section and said third chamber section comprise a first cross-section area and said second chamber section comprises a second cross-section area, wherein said second cross-section area is smaller than said first cross-section area. Said second chamber section comprises a bypass gas channel for receiving an inhalation gas. Said second valve body is connected to said opening of said ventilation limb via said bypass gas channel. Said second valve body may be connected to an individual ventilation gas supply and said ventilation gas may pass the bypass gas channel during inhalation and advantageously will support the patient's inhalation effort. Said second valve body may be branch connected to a ventilator of said ventilation apparatus.

Preferably said bypass gas channel comprises a venturi nozzle. Said venturi nozzle in said bypass gas channel controls the leakage gas flow by activating or deactivating the controllable valve in the opening of the main gas path.

Furthermore, the venturi nozzle in the bypass gas channel may control leakage gas in the bypass gas channel by augmenting the pressure differential across an opening (e.g. orifice) that communicates between the main gas path and the bypass gas channel.

Preferably the second chamber section comprises at least a second controllable leakage channel. Hence the at least second controllable leakage channel deactivates or weakens the leakage gas flow during inhalation by a gas pressure reduction in the bypass gas channel and activates the leakage gas flow during exhalation.

Further advantageous aspects of the invention are explained in the following by means of exemplary embodiments and the figures. In the drawings, it is shown in a schematic manner. Furthermore, a numeric counting within this application is just used to differ between said parts of said gas valve.
- Figure 1:: A first embodiment of a gas valve with a leakage channel with a first gas flow direction in a schematic view,
- Figure 1a:: said gas valve of Figure 1, showing the first cross-section area A1 in said gas valve, in a schematic view along VI
- Figure 1b:: said gas valve of Figure 1, showing the second cross-section area A2 in said gas valve, in a schematic view along VII
- Figure 2:: said gas valve of Figure 1 with the leakage channel with a second gas flow direction in a schematic view,
- Figure 3:: said gas valve of Figure 1 with a controllable valve in a schematic view,
- Figure 4:: said gas valve of Figure 1 with a venturi nozzle in the leakage channel in a schematic view,
- Figure 5:: said gas valve of Figure 1 with a filter media in the leakage channel in a schematic view,
- Figure 6:: A second embodiment of a gas valve with adjustable venturi nozzle (inactivated/deflated state) in a schematic view,
- Figure 7:: said gas valve of Figure 6 (activated/inflated state) in a schematic view,
- Figure 8:: a circuit comprising at least a gas valve of the previous mentioned Figures in a schematic view,
- Figure 9:: a first embodiment of a ventilator apparatus with a circuit comprising at least a gas valve of the previous mentioned Figures in a schematic view,
- Figure 10:: a second embodiment of a ventilator apparatus with a circuit comprising at least a gas valve connected adjacent to a limb in a schematic view,
- Figure 11:: Another embodiment of a gas valve with adjustable venturi nozzle (inactivated/deflated state) in a schematic view,
- Figure 12:: said gas valve of Figure 11 (activated/inflated state) in a schematic view, and
- Figure 13:: a third embodiment of a ventilator apparatus with a circuit comprising at least a gas valve of Figure 11 in a schematic view.

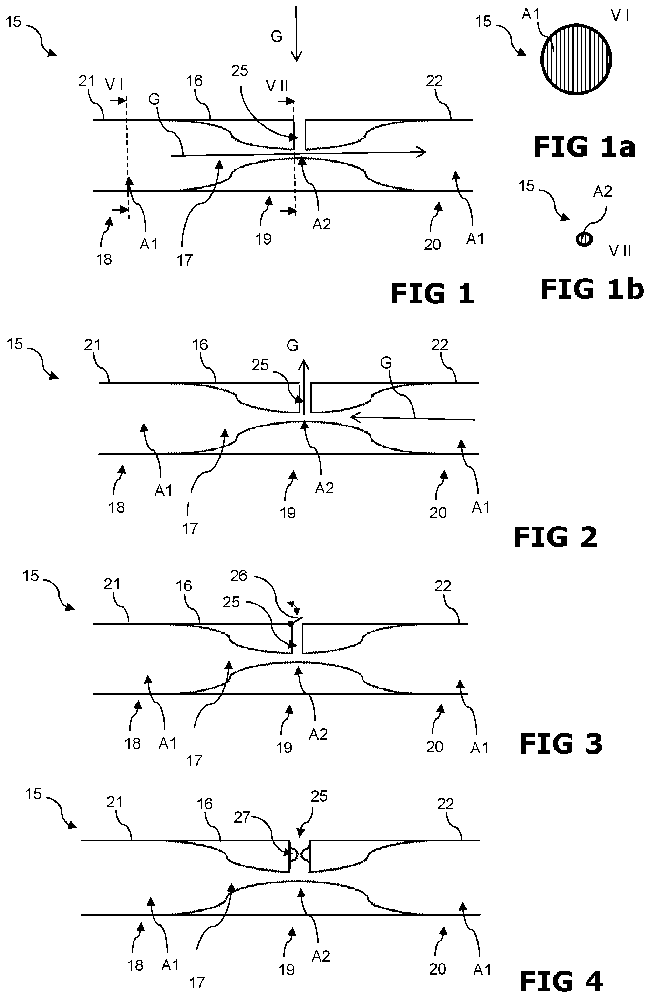

In the following detailed description, reference is made to the accompanying drawings Fig. 1 to Fig. 12, which form a part hereof, and in which are shown, by way of illustration, specific examples of embodiments in which the present disclosure may be practiced. These embodiments are described in sufficient detail to enable a person of ordinary skill in the art to practice the present disclosure. However, other embodiments may be utilized, and structural, system, and process changes may be made without departing from the scope of the disclosure. The following embodiments may be structurally and functionally mixed with each other to form another embodiment.

The following description may include examples to help enable one of ordinary skill in the art to practice the disclosed embodiments. The use of the terms "exemplary," "by example," and "for example," means that the related description is explanatory, and though the scope of the disclosure is intended to encompass the examples and legal equivalents, the use of such terms is not intended to limit the scope of an embodiment or this disclosure to the specified components, steps, features, functions, or the like.

The phrase "at least one of" when used with a list of items means different combinations of one or more of the listed items may be used and only one of each item in the list may be needed. For example, "at least one of item A, item B, and item C" may include, without limitation, item A or item A and item B. This example may also include item A, item B, and item C, or item B and item C. In other examples, "at least one of" may be, without limitation, two of item A, one of item B, and 10 of item C; four of item B and seven of item C; and other suitable combinations.

As used in this disclosure, any relational term, such as "first," "second," "over," "top," "bottom," "side," etc., is used for clarity and convenience in understanding the disclosure and accompanying drawings and does not connote or depend on any specific preference, orientation, or order, except where the context clearly indicates otherwise.

As used in this disclosure, the term "and/or" means and includes any and all combinations of one or more of the associated listed items.

When one component is "associated" with another component, the association is a physical association in these examples. For example, a first component may be considered to be associated with a second component by being secured to the second component by welding, fasteners or connected to the second component in some other suitable manner. The first component may also be connected to the second component using a third, intervening component by which the first component may also be considered to be associated with the second component.

The illustrations presented in this disclosure are not meant to be actual views of any particular system or device, but are merely idealized representations that are employed to describe the disclosed embodiments. Thus, the drawings are not necessarily to scale and relative dimensions may have been exaggerated for the sake of clarity. Additionally, elements common between figures may retain the same or similar numerical designation.

The following description provides specific details in order to provide a thorough description of embodiments of this disclosure. However, a person of ordinary skill in the art will understand that the embodiments of this disclosure may be practiced without employing these specific details.

The herein disclosed figures are illustrated as section schematic views - i.e. the actual shapes are three-dimensional (e.g. round or tubular).

Fig. 1 and Fig. 2 disclose a leakage gas valve 15 for a ventilation apparatus comprising a first valve body 16 having a main gas path 17 chamber for guiding an inhalation gas to a patient, comprising at least three chamber sections - a first chamber section 18, a second chamber section 19 and third chamber section 20 - an inlet duct 21 for supplying the ventilation gas G to the first chamber section 18 and an outlet duct 22 for releasing the ventilation gas from the third chamber section 20. Said first chamber section 18 and said third chamber section 20 comprise a first cross-section A1 area and said second chamber section comprises a second cross-section area A2. Said second chamber section 19 comprises a leakage channel 25 for allowing a leakage gas flow out of the gas valve 15 during exhalation. Said second cross-section area A2 is smaller than said first cross-section area A1 - see also Fig. 1a and Fig. 1b. The second chamber section 19 is limited by a continuous changing second cross-section area A2 along the main gas path, which changes its diameter from the diameter of the first cross-section A1 area to a minimum second cross-section area A2 and afterwards expands its diameter again to the diameter of the first cross-section area A1 in said third chamber section 20. Thus, a negative gas pressure is created in the main gas path due to the venturi effect, which prevents gas from leaking out of the leakage channel 25 during inhalation or may even draw a small amount of ambient gas into the gas valve through the leakage channel 15 during inhalation as shown in Fig. 1. The leakage channel 25 is a tubular channel and the leakage channel 25 is arranged at a minimum cross section area A2 at said second chamber section 19.

During exhalation, the gas flow from the ventilation apparatus to the patient is zero or reduced to a minimum (not shown). Hence no venturi effect occurs in said three chamber sections 18, 19, 20 of said gas valve 15 and the leakage gas flow is directed out of the leakage channel 25 of the gas valve 15, as shown in Fig. 2.

Fig. 3 shows the gas valve 15 according to Fig. 1 or Fig. 2, which in addition comprises controllable valve 26 arranged at the leakage channel 25 for controlling said leakage gas flow. Said controllable valve 26 is placed in the second chamber section 19 and is a very low opening pressure valve and the action of the negative pressure created during inhalation may keep or may pull the controllable valve 26 mechanically closed, while during exhalation it may open very easily for low work of breathing or low exhalation effort of the patient.

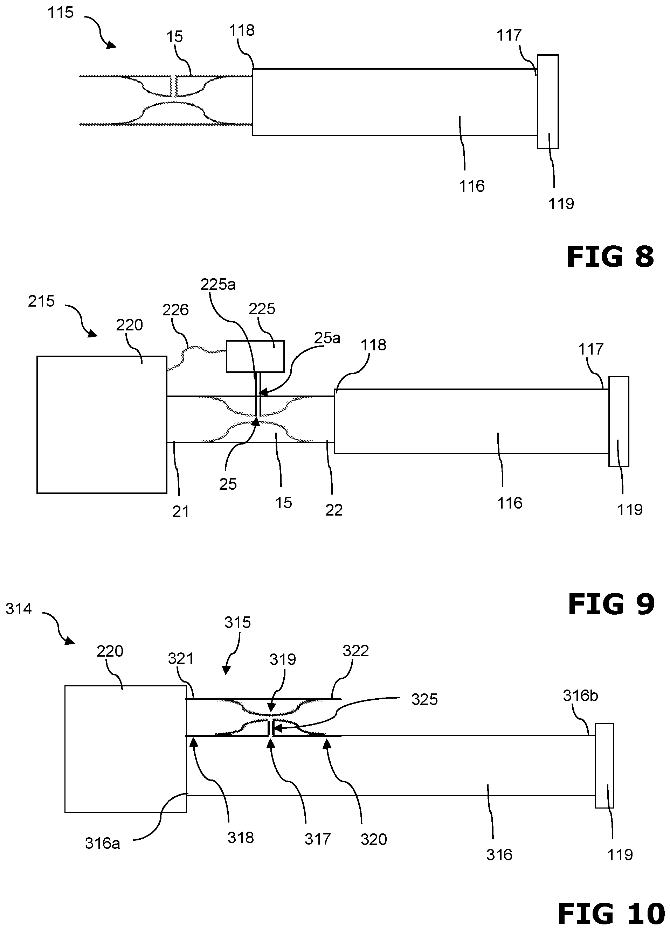

Fig. 4 shows the gas valve 15 according to one of the Fig. 1 to Fig 3, which in addition comprises a venturi nozzle 27 arranged in the leakage channel 25, which is used to measure the leakage gas flow during exhalation according to the principles of a venturi flowmeter concept. Such a gas valve 15 may be connected to a measuring device for measuring said leakage gas flow of said leakage channel 25 during exhalation - see Fig. 8. Said measuring device may use a pressure differential measurement sensor, especially a pressure differential measurement sensor of a ventilator apparatus. Said measuring device detects the exhalation behaviour of the patient during exhalation. Furthermore, said leakage channel 25 comprises at least one opening for connecting said measuring device for measuring said leakage gas flow. Said at least one opening may be connected with connections for measuring the pressure or the differential pressure in the leakage channel to determine the gas flow through the leakage channel 25 during inhalation and/or exhalation.

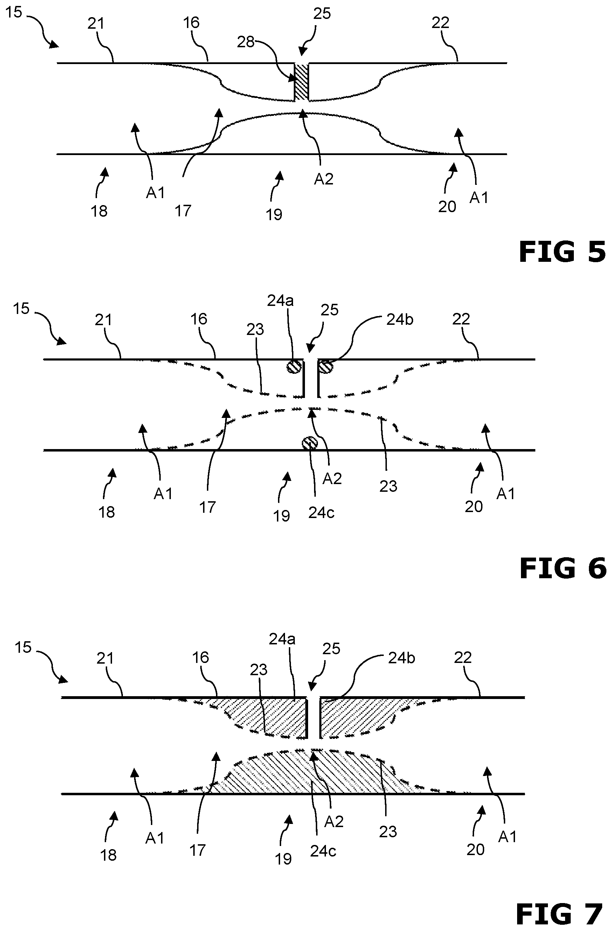

Fig. 5 shows the gas valve 15 according to one of the Fig. 1 to Fig. 4, which in addition comprises a filter medium 28 for filtering said leakage gas flow to filter inhaled and/or exhaled ventilation gas arranged in the leakage channel 25.

Fig. 6 und Fig. 7 show the gas valve 15 according to one of the Fig. 1 to Fig. 5, while the second cross-section area A2 of the second chamber section 19 is adjustable. As described above a venturi principle occurs within the main ventilation path of the gas valve 15. Adjusting the second cross-section area A2 of the second chamber section 19 is useful to match the gas flow rate in the main gas path by adjusting the venturi nozzle geometry in the main gas path. Furthermore, said second chamber section 19 comprises at least a first cage structure 23 with at three adjustable balloons 24a-24c. The first cage structure 23 forms the geometric structure of the venturi geometry within the main gas path - see Fig. 6. Said adjustable balloons 24a-24c may be inflated to fill out the cage structure - see. Fig. 7.

Said adjustable balloons 24a-24c comprise a controllable opening for connecting a fluid pump. Said adjustable balloons 24a-24c are connected to the said fluid pump, which inflate the balloons 24a-24c with a gas or a fluid. Furthermore, said balloons 24a-24c comprise a valve for deflating said balloons (not shown).

In another embodiment said second chamber section 19 comprises an adjustable chamber wall. The thickness of said chamber wall may be increased or decreased or the location of said chamber wall may be changed to adjust the second cross-section area in the second chamber section to produce a desired venturi geometry in the second chamber section (not shown).

Fig. 8 shows a circuit 115 for a ventilation system comprising a ventilation limb 116 and at least a gas valve 15 according to one of the above-mentioned gas valves. The ventilation limb 116 consists of a proximal end 117 and a distal end 118. The gas valve 15 is assembled at the distal end 118 of the ventilation limb 116. Said proximal end 117 of the ventilation limb 116 is connected to a respiration mask 119.

Fig. 9 shows a ventilation system 215 comprising a ventilator apparatus 220, a ventilation limb 116, and at least a gas valve 15 according to one of the above-mentioned gas valves. The gas valve 15 is connected to a pressure differential measurement sensor 225 or another flow sensor for measuring said leakage gas flow of said leakage channel 25 during exhalation. The leakage channel 25 comprises at least one opening 25a for connecting said pressure differential measurement sensor 225 for measuring said leakage gas flow. Said at least one opening 25a is connected with connections 225a for measuring the differential pressure in the leakage channel 25 to determine the gas flow through the leakage channel 25 during inhalation and/or exhalation. Said pressure differential measurement sensor 225 is electrically connected with the ventilator apparatus 220 via connections 226 to transmit the measured differential pressure data. Said pressure differential measurement sensor 225 may alternatively be located inside ventilation apparatus 220. The gas valve 15 is assembled at the ventilation apparatus 220 with the inlet duct 21 and is assembled at the distal end 118 of the ventilation limb 116 with the outlet duct 22. Said proximal end 117 of the ventilation limb 116 is connected to a respiration mask 119.

Fig. 10 shows a further embodiment of a ventilation system 314 comprising a ventilator apparatus 220 a ventilation limb 316 and at least a gas valve 315 according to one of the above-mentioned gas valves. The gas valve 315 comprises the same structural and functional features of one of the gas valves according to Fig. 1 to Fig. 7 and is determined as second valve body comprising a bypass gas channel 325 for receiving an inhalation gas. Said ventilation limb 316 comprises a leakage opening 317, e.g. a hole, a distal end 316a and a proximal end 316b. Said gas valve 315 comprises at least three chamber sections (first 318, second 319 and third chamber section 320), an inlet duct 321 for supplying the ventilation gas to the first chamber section 318 and an outlet duct 322 for releasing the ventilation gas from the third chamber section 320. Said first chamber section 318 and said third chamber section 320 comprise a first cross-section area and said second chamber section 319 comprises a second cross-section area, wherein said second cross-section area is smaller than said first cross-section area - see e.g. Fig. 1 or Fig. 2 above. Said second chamber section 319 comprises a bypass gas channel 325 for receiving an inhalation gas. Said second valve body is connected to said hole 317 of said ventilation limb 316 via said bypass gas channel 325. Said gas valve 315 is connected to a ventilation gas supply of said ventilation apparatus 220 with the inlet duct 321.

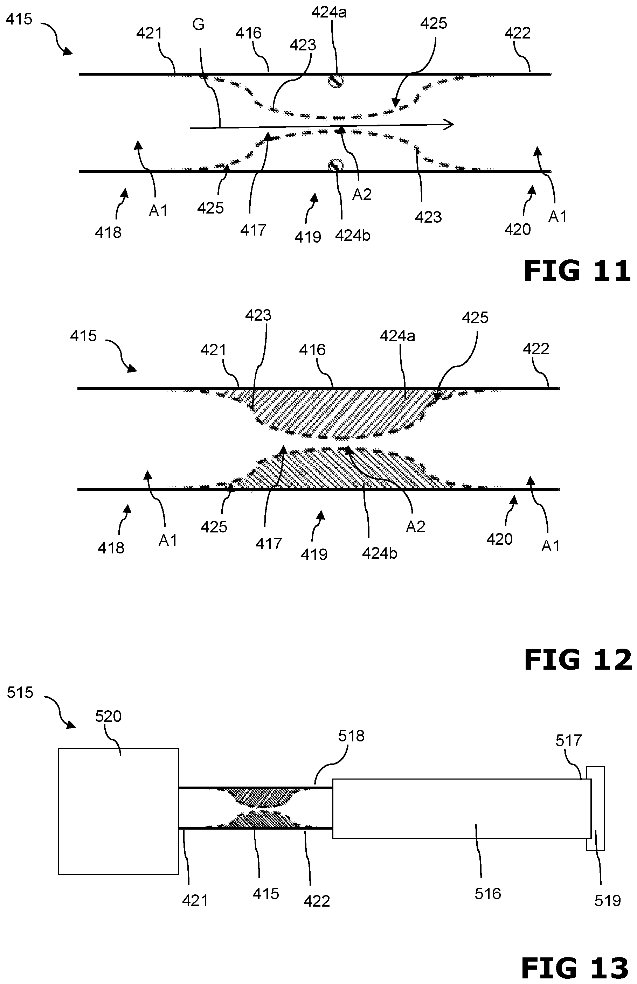

Fig. 11 and Fig. 12 disclose a gas valve 415 for a ventilation apparatus comprising a first valve body 416 having a main gas path 417 chamber for guiding an inhalation gas to a patient, comprising at least three chamber sections (first 418, second 419 and third chamber section 420), an inlet duct 421 for supplying the ventilation gas G to the first chamber section 418 and an outlet duct 422 for releasing the ventilation gas from the third chamber section 420. Said first chamber section 418 and said third chamber section 420 comprise a first cross-section A1 area and said second chamber section comprises a second cross-section area A2. Said second cross-section area A2 is smaller than said first cross-section area A1. The second chamber section 419 is limited by a continuously changing second cross-section area A2 along the main gas path, which changes its diameter from the diameter of the first cross-section A1 area to a minimum second cross-section area A2 and afterwards expands its diameter again to the diameter of the first cross-section area A1. The second cross-section area A2 of the second chamber section 419 is adjustable and said second chamber section comprises at least a cage structure 423 with adjustable balloons 424a, 424b. The venturi geometry/shape is created/controlled by the shape of the cage structure 423 comprising holes 425 for pass through of said gas flow. Therefore, when the balloons 424a, 424b are inflated, they will fill the cage structure 423 to form the venturi shape in the main gas path - see Fig. 12. In other words, said cage structure 423 is a three-dimensional grid structure, forming a contained volume or shape (e.g. venturi nozzle) which can be filled by an expandable member such as a balloon 424a, 424b.

Said balloons 424a, 424b are connected to a fluid pump or an adjustable reservoir. Said adjustable balloons 424a, 424b are connected to the said fluid pump, which inflate the balloons 424a, 424b with a gas or a fluid.

Said second chamber section may comprise a leakage channel for allowing a leakage gas flow out of the gas valve during exhalation, as described in the gas valve embodiments above (not shown).

Fig. 13 shows a ventilation system 515 comprising a ventilator apparatus 520 a ventilation limb 516 and at least a gas valve 415 according to the gas valves of Fig. 11 of Fig. 12. The gas valve 415 is assembled at the ventilation apparatus 520 with the inlet duct 421 and is assembled at the distal end 518 of the ventilation limb 516 with the outlet duct 422. Said proximal end 517 of the ventilation limb 516 is connected to a respiration mask 519.
- 15: leakage gas valve
- 16: first valve body
- 17: main gas path
- 18: first chamber section
- 19: second chamber sections
- 20: third chamber sections
- 21: inlet duct
- 22: outlet duct
- 24a-24c: Balloons (inflatable members)
- 25: leakage channel
- 25a: Opening in 25
- 26: controllable valve
- 27: venturi nozzle (valve)
- 28: filter medium

- 115: circuit
- 116: ventilation limb
- 117: proximal end of 116
- 118: distal end of 116
- 119: respiration mask

- 215: ventilation system
- 220: ventilator apparatus
- 225: pressure differential measurement sensor
- 226: connections

- 314: ventilation system
- 315: gas valve
- 316: ventilation limb
- 316a: distal end of 316
- 316b: proximal end of 316
- 317: leakage opening of 316
- 318: first chamber section
- 319: second chamber sections
- 320: third chamber sections
- 321: inlet duct
- 322: outlet duct
- 325: bypass gas channel

- 415: gas valve
- 416: first valve body
- 417: main gas path
- 418: first chamber section
- 419: second chamber sections
- 420: third chamber sections
- 421: inlet duct
- 422: outlet duct
- 423: cage structure
- 424a-424b: Balloons (inflatable members)
- 425: holes of 423

- 515: ventilation system
- 516: ventilation limb
- 517: proximal end of 516
- 518: distal end of 516
- 519: respiration mask
- 520: ventilator apparatus

- G: ventilation gas
- A1: first cross-section area
- A2: second cross-section area

## Claims

1. A leakage gas valve (15) for a ventilation apparatus comprising a first valve body (16) having a main gas path chamber (17) for guiding a ventilation gas to a patient for inhalation, comprising
• at least three chamber sections (first (18), second (19) and third chamber section (20)),
• an inlet duct (21) for supplying the ventilation gas to the first chamber section (18),
• an outlet duct (22) for releasing the ventilation gas from the third chamber section (20),
• wherein said first chamber section (18) and said third chamber section (20) comprise a first cross-section area (A1)
• and said second chamber section (19) comprises a second cross-section area (A2),
• wherein said second cross-section area (A2) is smaller than said first cross-section area (A1),
• said second chamber section (19) comprises a leakage channel (25) for allowing a leakage gas flow out of the gas valve (15) during exhalation.

2. The gas valve according to claim 1, wherein said leakage channel (25) comprises a controllable valve (26) for controlling said leakage gas flow, wherein said controllable valve (26) is preferably a gate-type valve.

3. The gas valve according to claim 1 or 2, wherein said leakage channel (25) is a tubular channel and preferably contains at least a venturi valve (27).

4. The gas valve according to any of the previous claims, wherein said leakage channel (25) is arranged at a minimum cross section area at said second chamber section (19).

5. The gas valve according to any of the previous claims, wherein a measuring device for measuring said leakage gas flow of said leakage channel (25) during exhalation is provided.

6. The gas valve according to claim 5, wherein said leakage channel (25) comprises at least one opening for connecting said measuring device for measuring said leakage gas flow.

7. The gas valve according to any of the previous claims, wherein said leakage channel (25) comprises at least a filter medium (28) for filtering said leakage gas flow.

8. The gas valve according to claims 1 to 7, wherein the second cross-section area (A2) of the second chamber section (19) is adjustable, while preferably said second chamber section (19) comprises an adjustable chamber wall.

9. The gas valve according to claims 1 to 7, wherein the second cross-section area (A2) of the second chamber section (19) is adjustable and said second chamber section (19) comprises at least a first cage structure (23) with at least one adjustable balloon (24a-24c).

10. The gas valve according to claim 9, wherein said at least one adjustable balloon comprises a controllable opening for connecting a fluid pump.

11. The gas valve according to any of the previous claims, wherein there is a second valve body (315) comprising a bypass gas channel (325) for receiving the ventilation gas, wherein said second valve body (315) is connected to said first valve body (16) by said leakage channel (25).

12. The gas valve according to claim 11, wherein said second valve body (315) comprises a venturi nozzle.

13. The gas valve according to any of the previous claims, wherein the second chamber section comprises at least a second controllable leakage channel.

14. A circuit (115) for a ventilation system (215; 315) comprising a ventilation limb (116) and at least a leakage gas valve (15) according to any of the previous claims, and the gas valve (15) is preferably assembled at a distal end (118) of the ventilation limb (116).

15. A circuit (115) for a ventilation system (215; 314) comprising a ventilation limb (116) and at least a leakage gas valve (15) according to any of the claims 1 to 13, and preferably the leakage gas valve is assembled at a proximal end (118) of the ventilation limb (116).

## Patentansprüche

1. Leckgasventil (15) für eine Beatmungsvorrichtung, umfassend einen ersten Ventilkörper (16) mit einer Hauptgaswegkammer (17) zum Leiten eines Beatmungsgases zu einem Patienten zum Einatmen, welches Folgendes umfasst
• mindestens drei Kammerabschnitte (erster (18), zweiter (19) und dritter Kammerabschnitt (20)),
• einen Einlasskanal (21) zum Zuführen des Beatmungsgases zum ersten Kammerabschnitt (18),
• einen Auslasskanal (22) zum Abgeben des Beatmungsgases aus dem dritten Kammerabschnitt (20),
• wobei der erste Kammerabschnitt (18) und der dritte Kammerabschnitt (20) eine erste Querschnittsfläche (A1) umfassen
• und der zweite Kammerabschnitt (19) eine zweite Querschnittsfläche (A2) aufweist,
• wobei die zweite Querschnittsfläche (A2) kleiner ist als die erste Querschnittsfläche (A1),
• der zweite Kammerabschnitt (19) einen Leckagekanal (25) aufweist, um während der Ausatmung einen Leckagegasstrom aus dem Gasventil (15) herauszulassen.

2. Gasventil nach Anspruch 1, wobei der Leckagekanal (25) ein steuerbares Ventil (26) zum Steuern des Leckgasstroms umfasst, wobei das steuerbare Ventil (26) vorzugsweise ein Absperrventil ist.

3. Gasventil nach Anspruch 1 oder 2, wobei der Leckagekanal (25) ein rohrförmiger Kanal ist und vorzugsweise mindestens ein Venturiventil (27) enthält.

4. Gasventil nach einem der vorherigen Ansprüche, wobei der Leckagekanal (25) an einer minimalen Querschnittsfläche an dem zweiten Kammerabschnitt (19) angeordnet ist.

5. Gasventil nach einem der vorherigen Ansprüche, wobei ein Messgerät zum Messen des Leckagegasstroms des Leckagekanals (25) während des Ausatmens vorgesehen ist.

6. Gasventil nach Anspruch 5, wobei der Leckagekanal (25) mindestens eine Öffnung zum Anschluss des Messgeräts zum Messen des Leckgasstroms umfasst.

7. Gasventil nach einem der vorstehenden Ansprüche, wobei der Leckagekanal (25) mindestens ein Filtermedium (28) zum Filtern des Leckgasstroms umfasst.

8. Gasventil nach den Ansprüchen 1 bis 7, wobei die zweite Querschnittsfläche (A2) des zweiten Kammerabschnitts (19) einstellbar ist, wobei vorzugsweise der zweite Kammerabschnitt (19) eine einstellbare Kammerwand umfasst.

9. Gasventil nach einem der Ansprüche 1 bis 7, wobei die zweite Querschnittsfläche (A2) des zweiten Kammerabschnitts (19) einstellbar ist und der zweite Kammerabschnitt (19) mindestens eine erste Käfigstruktur (23) mit mindestens einem einstellbaren Ballon (24a-24c) umfasst.

10. Gasventil nach Anspruch 9, wobei der mindestens eine einstellbare Ballon eine steuerbare Öffnung zum Anschluss einer Flüssigkeitspumpe aufweist.

11. Gasventil nach einem der vorhergehenden Ansprüche, wobei ein zweiter Ventilkörper (315) mit einem Bypass-Gaskanal (325) zur Aufnahme des Beatmungsgases vorgesehen ist, wobei der zweite Ventilkörper (315) über den Leckagekanal (25) mit dem ersten Ventilkörper (16) verbunden ist.

12. Gasventil nach Anspruch 11, wobei der zweite Ventilkörper (315) eine Venturidüse umfasst.

13. Gasventil nach einem der vorherigen Ansprüche, wobei der zweite Kammerabschnitt mindestens einen zweiten steuerbaren Leckagekanal umfasst.

14. Eine Schaltung (115) für ein Beatmungssystem (215; 315), die einen Beatmungszweig (116) und mindestens ein Leckgasventil (15) gemäß einem der vorherigen Ansprüche umfasst, wobei das Gasventil (15) vorzugsweise an einem distalen Ende (118) des Beatmungszweigs (116) angebracht ist.

15. Eine Schaltung (115) für ein Beatmungssystem (215; 314), die einen Beatmungszweig (116) und mindestens ein Leckgasventil (15) gemäß einem der Ansprüche 1 bis 13 umfasst, wobei das Leckgasventil vorzugsweise an einem proximalen Ende (118) des Beatmungszweigs (116) angebracht ist**.**

## Revendications

1. Soupape de gaz de fuite (15) pour un appareil de ventilation comprenant un premier corps de soupape (16) ayant une chambre de passage de gaz principale (17) permettant de guider un gaz de ventilation vers un patient pour inhalation, comprenant
• au moins trois sections de chambre (première (18), deuxième (19) et troisième section de chambre (20)),
• un conduit d'entrée (21) permettant de fournir le gaz de ventilation à la première section de chambre (18),
• un conduit de sortie (22) permettant d'évacuer le gaz de ventilation de la troisième section de chambre (20),
• dans laquelle ladite première section de chambre (18) et ladite troisième section de chambre (20) comprennent une première zone de section transversale (A1)
• et ladite deuxième section de chambre (19) comprend une deuxième zone de section transversale (A2),
• dans laquelle ladite deuxième zone de section transversale (A2) est plus petite que ladite première zone de section transversale (A1),
• ladite deuxième section de chambre (19) comprend un canal de fuite (25) permettant à un flux de gaz de fuite de s'échapper de la soupape de gaz (15) pendant l'exhalation.

2. Soupape de gaz selon la revendication 1, dans laquelle ledit canal de fuite (25) comprend une soupape contrôlable (26) permettant de contrôler ledit flux de gaz de fuite, ladite soupape contrôlable (26) est de préférence une soupape à guillotine.

3. Soupape de gaz selon la revendication 1 ou 2, dans laquelle ledit canal de fuite (25) est un canal tubulaire et contient de préférence au moins une soupape de type venturi (27).

4. Soupape de gaz selon l'une quelconque des revendications précédentes, dans laquelle ledit canal de fuite (25) est disposé à une zone de section transversale minimale au niveau de ladite deuxième section de chambre (19).

5. Soupape de gaz selon l'une quelconque des revendications précédentes, dans laquelle un dispositif de mesure permettant de mesurer ledit flux de gaz de fuite dudit canal de fuite (25) pendant l'expiration est prévu.

6. Soupape de gaz selon la revendication 5, dans laquelle ledit canal de fuite (25) comprend au moins une ouverture permettant de raccorder ledit dispositif de mesure permettant de mesurer ledit flux de gaz de fuite.

7. Soupape de gaz selon l'une quelconque des revendications précédentes, dans laquelle ledit canal de fuite (25) comprend au moins un média filtrant (28) permettant de filtrer ledit flux de gaz de fuite.

8. Soupape de gaz selon les revendications 1 à 7, dans laquelle la deuxième zone de section transversale (A2) de la deuxième section de chambre (19) est réglable, tandis que de préférence ladite deuxième section de chambre (19) comprend une paroi de chambre réglable.

9. Soupape de gaz selon les revendications 1 à 7, dans laquelle la deuxième zone de section transversale (A2) de la deuxième section de chambre (19) est réglable et ladite deuxième section de chambre (19) comprend au moins une première structure de cage (23) avec au moins un ballon réglable (24a-24c).

10. Soupape de gaz selon la revendication 9, dans laquelle ledit au moins un ballon réglable comprend une ouverture contrôlable permettant de raccorder une pompe à fluide.

11. Soupape de gaz selon l'une quelconque des revendications précédentes, dans laquelle il y a un deuxième corps de soupape (315) comprenant un canal de gaz de dérivation (325) permettant de recevoir le gaz de ventilation, dans laquelle ledit deuxième corps de soupape (315) est raccordé audit premier corps de soupape (16) par ledit canal de fuite (25).

12. Soupape de gaz selon la revendication 11, dans laquelle ledit deuxième corps de soupape (315) comprend une buse de type venturi.

13. Soupape de gaz selon l'une quelconque des revendications précédentes, dans laquelle la deuxième section de chambre comprend au moins un deuxième canal de fuite contrôlable.

14. Circuit (115) pour un système de ventilation (215 ; 315) comprenant un conduit de ventilation (116) et au moins une soupape de gaz de fuite (15) selon l'une quelconque des revendications précédentes, et la soupape de gaz (15) est de préférence assemblée à une extrémité distale (118) du conduit de ventilation (116).

15. Circuit (115) pour un système de ventilation (215 ; 314) comprenant un conduit de ventilation (116) et au moins une soupape de gaz de fuite (15) selon l'une quelconque des revendications 1 à 13, et de préférence la soupape de gaz de fuite est assemblée à une extrémité proximale (118) du conduit de ventilation (116).
